# EUROPEAN PATENT APPLICATION

(11) **EP 3 536 309 A1**
(43) Date of publication of application: **11.09.2019**
(21) Application number: 19160961.9
(22) Date of filing: 06.03.2019
(51) Int. Cl.: A61K 9/00, A61K 47/32, A61K 9/08, A61K 9/19, A61K 31/00

(54) **STERILE INJECTABLE COMPOSITION COMPRISING CARFILZOMIB**

(30) Priority: 06.03.2018 IN 201821008249
(71) Applicant: Cadila Healthcare Limited, Ahmedabad 380 015 Gujarat (IN)
(72) Inventor: MUTHAIYYAN, Kannan Essakimuthu, 382210 Ahmedabad (IN); SINGH, Debjani Manoj, 382210 Ahmedabad (IN); KHATRI, Nirav Ishwarlal, 382210 Ahmedabad (IN); SHARMA, Omprakash Ganesh Ram, 382210 Ahmedabad (IN)
(74) Representative: Gill Jennings & Every LLP

(57) **Abstract**

The present invention relates to sterile injectable compositions comprising carfilzomib and one or more phospholipids. The present invention also relates to processes for preparing the sterile injectable compositions.

## Description

### Field of the invention

The field of the present invention relates to sterile injectable compositions comprising carfilzomib and one or more phospholipids. The present invention also relates to processes for preparing the sterile injectable compositions.

### Background of the invention

Cancer is a group of diseases involving abnormal cell growth with the potential to invade or spread to other parts of the body. Chemotherapy is a category of cancer treatment that uses at least one anti-cancer drug (chemotherapeutic agent) as part of a standardized chemotherapy regimen.

Carfilzomib is a modified tetrapeptidyl epoxide. The chemical name for carfilzomib is (2S)-N-((S)- 1-((S)-4-methyl-1-((R)-2-methyloxiran2-yl)-1-oxopentan-2-ylcarbamoyl)-2-phenylethyl)-2-((S)-2-(2-morpholinoacetamido)-4-phenylbutanamido)-4-methylpentanamide. Carfilzomib is practically insoluble in water and very slightly soluble in acidic conditions. Carfilzomib is indicated for the treatment of Relapsed or Refractory Multiple Myeloma. The marketed product Kyprolis®, of Onyx Therapeutics Inc., a wholly owned subsidiary of Amgen Inc., is a sterile, white to off-white lyophilized powder and is available as a single-dose 30 mg or 60 mg vial. Each 30 mg vial contains 30 mg of carfilzomib, 1500 mg sulfobutylether beta-cyclodextrin, and 28.9 mg anhydrous citric acid and sodium hydroxide for pH adjustment (target pH 3.5) and each 60 mg vial contains 60 mg of carfilzomib, 3000 mg sulfobutylether beta-cyclodextrin, 57.7 mg citric acid, and sodium hydroxide for pH adjustment (target pH 3.5).

The effect of sulfobutylether beta-cyclodextrin on the kidney appears to be dose-dependent and temporally determined. In animal trials, single-dose studies up to 2000 mg/kg i.v. sulfobutylether beta-cyclodextrin showed renal tolerance. Evidence of renal cellular toxicity was observed in studies with repeated administration over 1-6 months at sulfobutylether beta-cyclodextrin doses of ≥160 mg/kg/day, although even at much higher doses renal function was not impaired (Luke et al., J Pharm Sci 2010; 99: 3291-3301). Marc A von Mach et al. (BMC Clin Pharmacol. 2006; 6: 6) mentions that there are chances of accumulation of sulphobutylether beta-cyclodextrin sodium in patients. It further mentions that systematic clinical investigations are required to prevent potential harm to patients due to an accumulation of sulphobutylether beta-cyclodextrin sodium.

There exists a need of an alternative, improved and stable injectable composition which enables efficacious and safe parenteral delivery of carfilzomib.

### Summary of the invention

In one general aspect, the present invention provides a sterile injectable composition comprising carfilzomib and one or more pharmaceutically acceptable excipients.

In another general aspect, the sterile injectable composition comprises carfilzomib, one or more phospholipids and one or more stabilizers.

In another general aspect, the sterile injectable composition may be in the form of a clear solution comprising carfilzomib-phospholipid micelles. The micelles may be carfilzomib-mPEG₂₀₀₀-DSPE micelles and/or carfilzomib-DMPG micelles.

In another general aspect, the sterile injectable composition may be in the form of a stable lyophilized powder comprising carfilzomib and one or more phospholipids.

In another general aspect, the present invention provides a process for preparing the sterile injectable composition.

In another general aspect, the invention provides a method of treating cancer by administering the sterile injectable composition of the present invention to the individual in need thereof.

The details of one or more embodiments of the invention are set forth in the description below. Other features, objects and advantages of the invention will be apparent from the description.

### Detailed description of the invention

The present invention provides a sterile injectable composition comprising carfilzomib and one or more pharmaceutically acceptable excipients.

The sterile injectable composition of the present invention may be in the form of a stable lyophilized powder or a stable, clear, aqueous solution. The sterile injectable composition of the invention is not a powder for suspension, liquid suspension or nanoparticulate dispersion.

The sterile injectable composition of the present invention may be administered via parenteral route, for example, intravenous (I.V.), subcutaneous (S.C.) or intramuscular (I.M.).

The sterile injectable composition of the present invention may provide one or more advantages like, providing a safe composition by solubilizing the drug without usage of ingredients such as sulfobutylether beta-cyclodextrin, polysorbate-80 or cremophor®, providing a stable, clear, aqueous solution suitable for I.V. administration compatible with routinely used i.v. sets and having improved stability, efficacy, and safety profile. Polysorbate-80 and cremophor are reported as the cause of hypersensitivity reactions while beta-cyclodextrin derivatives have been known to cause certain unwanted effects.

The sterile injectable composition of the present invention does not comprise cholesterol or cholesterol derivatives (for example, sodium cholesteryl sulphate etc.), lecithin or its derivative, phosphatidylcholines and/or polysorbate-80.

The sterile injectable composition may comprise carfilzomib and one or more phospholipids. The composition may comprise carfilzomib, 1,2-dimyristoyl-sn-glycero-3-phosphorylglycerol (DMPG), N-(carbonyl-methoxypolyethyleneglycol 2000)-1,2-distearoyl-sn-glycero-3-phosphoethanolamine (mPEG₂₀₀₀-DSPE), or any combination thereof. The composition may further comprise one or more stabilizers.

The stable, clear, aqueous solution may comprise carfilzomib, one or more phospholipids and one or more stabilizers. The stable, clear, aqueous solution may comprise drug micelles and is lyophilizable. The stable, clear, aqueous solution of the present invention does not comprise bilayered micelles (bicelles).

In one embodiment, the sterile injectable composition is a stable lyophilized powder comprising carfilzomib and one or more pharmaceutically acceptable excipients. The stable lyophilized powder may comprise carfilzomib, one or more phospholipids and one or more stabilizers.

In one embodiment, the sterile injectable composition is a stable, clear, aqueous solution comprising carfilzomib and one or more pharmaceutically acceptable excipients. The stable lyophilized powder may comprise carfilzomib, one or more phospholipids and one or more stabilizers.

In one embodiment, the invention provides a sterile injectable composition comprising carfilzomib which is therapeutically equivalent to the commercially available carfilzomib formulation marketed under the trade name Kyprolis®.

The sterile injectable composition comprising carfilzomib of the invention may provide value of AUC (area under the curve for a plot of concentration of drug in plasma vs. time) between 100 ng.h/mL and 700 ng.h/mL, for example, between 150 ng.h/mL and 650 ng.h/mL, between 200 ng.h/mL and 600 ng.h/mL, between 250 ng.h/mL and 550 ng.h/mL, between 300 ng.h/mL and 500 ng.h/mL, between 350 ng.h/mL and 450 ng.h/mL, 360 ng.h/mL, 370 ng.h/mL, 390 ng.h/mL, 410 ng.h/mL, 420 ng.h/mL or 430 ng.h/mL, when the sterile injectable composition comprising carfilzomib is administered as intravenous infusion to a human at a dose of 27 mg carfilzomib/m² in 2-10 minutes.

The sterile injectable composition comprising carfilzomib of the invention may provide value of AUC (area under the curve for a plot of concentration of drug in plasma vs. time) between 500 ng.h/mL and 1500 ng.h/mL, for example, between 600 ng.h/mL and 1400 ng.h/mL, between 700 ng.h/mL and 1300 ng.h/mL, between 750 ng.h/mL and 1250 ng.h/mL, between 800 ng.h/mL and 1200 ng.h/mL, between 850 ng.h/mL and 1150 ng.h/mL, 900 ng.h/mL, 925 ng.h/mL, 950 ng.h/mL, 1000 ng.h/mL, 1050 ng.h/mL or 1100 ng.h/mL, when the sterile injectable composition comprising carfilzomib is administered as intravenous infusion to a human at a dose of 56 mg carfilzomib/m² in 30 minutes.

The sterile injectable composition comprising carfilzomib of the invention may provide value of mean Cₘₐₓ (maximum concentration of drug in plasma, achieved after administration) between 2000 ng/mL and 6000 ng/mL, for example, between 2500 ng/mL and 5500 ng/mL, between 3000 ng/mL and 5000 ng/mL, between 3500 ng/mL and 4500 ng/mL, between 3600 ng/mL and 4400 ng/mL, between 3700 ng/mL and 4300 ng/mL or between 3800 ng/mL and 4250 ng/mL, when the sterile injectable composition comprising carfilzomib is administered as intravenous infusion to a human at a dose of 27 mg carfilzomib/m² in 2-10 minutes.

The sterile injectable composition comprising carfilzomib of the invention may provide value of mean Cₘₐₓ (maximum concentration of drug in plasma, achieved after administration) between 1000 ng/mL and 3000 ng/mL, for example, between 1100 ng/mL and 2900 ng/mL, between 1200 ng/mL and 2800 ng/mL, between 1300 ng/mL and 2700 ng/mL, between 1400 ng/mL and 2600 ng/mL, between 1500 ng/mL and 2500 ng/mL, between 1600 ng/mL and 2400 ng/mL, between 1700 ng/mL and 2300 ng/mL, between 1800 ng/mL and 2200 ng/mL or between 1900 ng/mL and 2100 ng/mL, when the sterile injectable composition comprising carfilzomib is administered as intravenous infusion to a human at a dose of 56 mg carfilzomib/m² in 30 minutes.

In one embodiment, the stable lyophilized powder for injection comprises carfilzomib and one or more phospholipids.

In another embodiment, the stable lyophilized powder for injection comprises carfilzomib and one or more micelle forming phospholipids, wherein the phospholipid is having fatty acid carbon chain length of 16 carbons or less, for example, 16 carbons, 14 carbons or 12 carbons.

In another embodiment, the stable lyophilized powder for injection comprises carfilzomib and one or more phospholipids. The stable lyophilized powder for injection may comprise carfilzomib and 1, 2-dimyristoyl-sn-glycero-3-phosphorylglycerol (DMPG). The stable lyophilized powder may further comprise one or more stabilizers.

In another embodiment, the stable lyophilized powder for injection comprises carfilzomib and N-(Carbonyl-methoxypolyethyleneglycol 2000)-1,2-distearoyl-sn-glycero-3-phosphoethanolamine (mPEG₂₀₀₀-DSPE). The stable lyophilized powder may further comprise polyvinylpyrrolidone.

In one embodiment, there is provided a vial comprising stable lyophilized powder comprising about 30 mg carfilzomib, about 90 mg N-(Carbonyl-methoxypolyethyleneglycol 2000)-1,2-distearoyl-sn-glycero-3-phosphoethanolamine sodium (mPEG₂₀₀₀-DSPE Na), about 525 mg polyvinylpyrrolidone K12 (PVP K12) and about 30 mg citric acid.

In another embodiment, there is provided a vial comprising stable lyophilized powder comprising about 60 mg carfilzomib, about 180 mg N-(Carbonyl-methoxypolyethyleneglycol 2000)-1,2-distearoyl-sn-glycero-3-phosphoethanolamine sodium (mPEG₂₀₀₀-DSPE Na), about 1050 mg polyvinylpyrrolidone K12 (PVP K12) and about 60 mg citric acid.

The amount of mPEG₂₀₀₀-DSPE present in a vial comprising stable lyophilized powder comprising carfilzomib may be between about 80 mg and about 200 mg, for example, about 90 mg, about 100 mg, about 110 mg, about 120 mg, about 130 mg, about 140 mg, about 150 mg, about 160 mg, about 170 mg, about 180 mg or about 190 mg. The weight ratio of carfilzomib to mPEG₂₀₀₀-DSPE may be between 2:1 and 1:5, for example, between 1:1 and 1:4, about 1:1, about 1:2, about 1:2.5, about 1:3 or about 1:4. The amount of PVP present in a vial comprising stable lyophilized powder comprising carfilzomib may be between about 100 mg and about 2000 mg, for example, about 200 mg, about 300 mg, about 400 mg, about 500 mg, about 525 mg, about 600 mg, about 700 mg, about 800 mg, about 900 mg, about 1000 mg, about 1050 mg, about 1100 mg, about 1200 mg, about 1300 mg, about 1400 mg, about 1500 mg, about 1600 mg, about 1700 mg, about 1800 mg or about 1900 mg. The weight ratio of carfilzomib to PVP may be between 1:1 and 1:30, for example, between 1:5 and 1:20, about 1:5, about 1:8.5, about 1:10, about 1:15, about 1:17.5, about 1:20 or about 1:25.

In one embodiment, the stable lyophilized powder for injection comprises carfilzomib, 1,2-dimyristoyl-sn-glycero-3-phosphorylglycerol (DMPG) and N-(Carbonyl-methoxypolyethyleneglycol 2000)-1,2-distearoyl-sn-glycero-3-phosphoethanolamine (mPEG₂₀₀₀-DSPE). The stable lyophilized powder may further comprise polyvinylpyrrolidone.

In one embodiment, there is provided a vial comprising stable lyophilized powder comprising about 30 mg carfilzomib, about 150 mg 1,2-dimyristoyl-sn-glycero-3-phosphorylglycerol sodium (DMPG Na), about 52.5 mg N-(Carbonyl-methoxypolyethyleneglycol 2000)-1,2-distearoyl-sn-glycero-3-phosphoethanolamine sodium (mPEG₂₀₀₀-DSPE Na), about 600 mg polyvinylpyrrolidone K12 (PVP K12) and about 5.25 mg citric acid.

In another embodiment, there is provided a vial comprising stable lyophilized powder comprising about 60 mg carfilzomib, about 300 mg 1,2-dimyristoyl-sn-glycero-3-phosphorylglycerol sodium (DMPG Na), about 105 mg N-(Carbonyl-methoxypolyethyleneglycol 2000)-1,2-distearoyl-sn-glycero-3-phosphoethanolamine sodium (mPEG₂₀₀₀-DSPE Na), about 1200 mg polyvinylpyrrolidone K12 (PVP K12) and about 10.5 mg citric acid.

In another embodiment, there is provided a vial comprising stable lyophilized powder comprising about 10 mg carfilzomib, about 1.75 mg 1,2-dimyristoyl-sn-glycero-3-phosphorylglycerol sodium (DMPG Na), about 30 mg N-(Carbonyl-methoxypolyethyleneglycol 2000)-1,2-distearoyl-sn-glycero-3-phosphoethanolamine sodium (mPEG₂₀₀₀-DSPE Na), about 85 mg polyvinylpyrrolidone K12 (PVP K12), 125 mg sucrose and about 4.25 mg citric acid (anhydrous).

In another embodiment, there is provided a vial comprising stable lyophilized powder comprising about 30 mg carfilzomib, about 5.25 mg 1,2-dimyristoyl-sn-glycero-3-phosphorylglycerol sodium (DMPG Na), about 90 mg N-(Carbonyl-methoxypolyethyleneglycol 2000)-1,2-distearoyl-sn-glycero-3-phosphoethanolamine sodium (mPEG₂₀₀₀-DSPE Na), about 255 mg polyvinylpyrrolidone K12 (PVP K12), about 375 mg sucrose and about 12.75 mg citric acid (anhydrous).

In another embodiment, there is provided a vial comprising stable lyophilized powder comprising about 60 mg carfilzomib, about 10.5 mg 1,2-dimyristoyl-sn-glycero-3-phosphorylglycerol sodium (DMPG Na), about 180 mg N-(Carbonyl-methoxypolyethyleneglycol 2000)-1,2-distearoyl-sn-glycero-3-phosphoethanolamine sodium (mPEG₂₀₀₀-DSPE Na), about 510 mg polyvinylpyrrolidone K12 (PVP K12), about 750 mg sucrose and about 25.5 mg citric acid (anhydrous).

The amount of DMPG present in a vial comprising stable lyophilized powder comprising carfilzomib may be between about 1 mg and about 500 mg, for example, about 1.5 mg, about 1.75 mg, about 2 mg, about 5 mg, about 10 mg, about 25 mg, about 50 mg, about 75 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg or about 320 mg. The weight ratio of carfilzomib to DMPG may be between 10:1 and 1:10, for example, between 6:1 and 1:6, about 9:1, about 8:1, about 7:1, about 6:1, about 5.7:1, about 5:1, about 4:1, about 3:1, about 2:1, about 1:1, about 1:2, about 1:3, about 1:4, about 1:5, about 1:6, about 1:7, about 1:8 or about 1:9.

The amount of mPEG₂₀₀₀-DSPE present in a vial comprising stable lyophilized powder comprising carfilzomib may be between about 10 mg and about 200 mg, for example, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg or about 190 mg. The weight ratio of carfilzomib to mPEG₂₀₀₀-DSPE may be between 2:1 and 1:5, for example, between 1:1 and 1:4, about 1:1, about 1:2, about 1:2.5, about 1:3 or about 1:4.

The amount of sucrose present in a vial comprising stable lyophilized powder comprising carfilzomib may be between about 50 mg and about 1500 mg, for example, about 100 mg, about 125 mg, about 150 mg, about 200 mg, about 250 mg, about 300 mg, about 350 mg, about 375 mg, about 400 mg, about 500 mg, about 600 mg, about 700 mg, about 750 mg, about 800 mg, about 900 mg, about 1000 mg, about 1100 mg, about 1200 mg, about 1300 mg or about 1400 mg. The weight ratio of carfilzomib to sucrose may be between 1:5 and 1:23, for example, about 1:10, about 1:12.5, about 1:15, about 1:20 or about 1:25. The amount of PVP present in a vial comprising stable lyophilized powder comprising carfilzomib may be between about 10 mg and about 2000 mg, for example, about 25 mg, about 50 mg, about 85 mg, about 100 mg, about 200 mg, about 255 mg, about 300 mg, about 400 mg, about 500 mg, about 510 mg, about 550 mg, about 600 mg, about 700 mg, about 800 mg, about 900 mg, about 1000 mg, about 1050 mg, about 1100 mg, about 1200 mg, about 1300 mg, about 1400 mg, about 1500 mg, about 1600 mg, about 1700 mg, about 1800 mg or about 1900 mg. The weight ratio of carfilzomib to PVP may be between 1:1 and 1:30, for example, about 1:5, about 1:8.5, about 1:10, about 1:15, about 1:17, about 1:20, about 1:22 or about 1:25.

In one embodiment, the stable lyophilized powder is powder for solution. The stable lyophilized powder may be reconstituted using an appropriate quantity of aqueous vehicle (e.g. water for injection, 5 % dextrose or 0.9% NaCl) to provide a stable, clear, aqueous reconstituted solution. Time to prepare the reconstituted solution may be referred as reconstitution time which may be not more than 15 minutes, for example, not more than 10 minutes or not more than 5 minutes. The reconstituted solution may be diluted using an appropriate quantity of diluent (e.g. water for injection, 5 % dextrose or 0.9% NaCl) to provide a stable, clear, aqueous diluted solution.
In one embodiment, the sterile injectable composition is a stable, clear, aqueous solution comprising one or more drugs and one or more phospholipids. The solution of the invention does not form any precipitate after storage for relevant time period, for example, for 6 hours, for 8 hours, for 10 hours, for 12 hours, for 18 hours, for 24 hours or for 48 hours, at 2°C -8°C temperature or at 25°C ± 2°C temperature. The stable, clear, aqueous solution of the invention does not comprise liposome or nanoparticle or any other particulate drug delivery system.

In another embodiment, the sterile injectable composition is a stable, clear, aqueous solution comprising one or more drugs, one or more phospholipids and one or more stabilizers.

In another embodiment, the stable, clear, aqueous solution comprises carfilzomib and one or more phospholipids. The stable, clear, aqueous solution may comprises carfilzomib, 1,2-dimyristoyl-sn-glycero-3-phosphorylglycerol (DMPG) and/or N-(Carbonyl-methoxypolyethyleneglycol 2000)-1,2-distearoyl-sn-glycero-3-phosphoethanolamine (mPEG₂₀₀₀-DSPE). The stable, clear, aqueous solution may further comprise polyvinylpyrrolidone. The solution may be lyophilizable. The solution may be used for parenteral administration. The solution for parenteral administration may be in the form of 100% water based solution and does not comprise any other solvent or liquid vehicle.

In another embodiment, the present invention provides a stable, clear, aqueous solution comprising carfilzomib, mPEG₂₀₀₀-DSPE, and polyvinylpyrrolidone, wherein the solution may have pH between about 2 and about 7, for example, about 3, about 3.5 or about 4.

In another embodiment, the present invention provides a stable, clear, aqueous solution comprising carfilzomib, DMPG, mPEG₂₀₀₀-DSPE and polyvinylpyrrolidone, wherein the solution may have pH between about 2 and about 7, for example, about 3, about 3.5 or about 4.

In another embodiment, the present invention provides a stable, clear, aqueous solution comprising carfilzomib, DMPG, mPEG₂₀₀₀-DSPE and one or more stabilizers, wherein the concentration of carfilzomib may be between about 0.05 mg/mL and about 50 mg/mL, for example, between about 0.25 mg/mL and about 10 mg/mL, about 1 mg/mL, about 2 mg/mL, about 5 mg/mL or about 8 mg/mL. The amount of DMPG present in the diluted solution comprising carfilzomib may be between about 0.01 mg/mL and about 10 mg/mL, for example, about 0.05 mg/mL, about 0.1 mg/mL, about 0.5 mg/mL, about 1 mg/mL, about 2 mg/mL, about 3 mg/mL, about 4 mg/mL, about 5 mg/mL, about 6 mg/mL, about 7 mg/mL, about 8 mg/mL or about 9 mg/mL. The amount of PVP present in the diluted solution comprising carfilzomib may be between about 1 mg/mL and about 100 mg/mL, for example, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 80 mg/mL, about 85 mg/mL, about 90 mg/mL or about 95 mg/mL.

In another embodiment, the present invention provides a stable, clear, aqueous solution comprising carfilzomib, mPEG₂₀₀₀-DSPE and one or more stabilizers, wherein the concentration of carfilzomib may be between about 0.05 mg/mL and about 50 mg/mL, for example, between about 0.25 mg/mL and about 10 mg/mL, about 1 mg/mL, about 2 mg/mL, about 5 mg/mL or about 8 mg/mL. The amount of mPEG₂₀₀₀-DSPE present in the diluted solution comprising carfilzomib may be between about 1 mg/mL and about 10 mg/mL, for example, about 2 mg/mL, about 3 mg/mL, about 4 mg/mL, about 5 mg/mL, about 6 mg/mL, about 7 mg/mL, about 8 mg/mL or about 9 mg/mL. The amount of PVP present in the diluted solution comprising carfilzomib may be between about 1 mg/mL and about 100 mg/mL, for example, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 80 mg/mL, about 85 mg/mL, about 90 mg/mL or about 95 mg/mL.

The stable, clear, aqueous solution may be in the form of micellar solution comprising carfilzomib, one or more phospholipids and one or more stabilizers.

### Definitions:

As used herein, and unless otherwise specified, the term "stable" refers to the stability of the product or the composition comprising one or more drugs having sufficient physical and chemical stability for a relevant period of time under the specified storage conditions.

### The term "physical stability" with respect to clear solution refers to maintenance of clear state without any drug precipitation, maintenance of color or colorless state and/or maintenance of dissolved oxygen level. The term "physical stability" with respect to lyophilized powder refers to maintenance of aesthetic appearance, absence of agglomerates, and maintenance of flowability, dispersibility and water content.

### The term "chemical stability" relates to maintenance of drug-related impurities in terms of total impurities, known impurities, single maximum known impurity and single maximum unknown impurity, within the allowed limits by the regulatory agency.

Unless and otherwise mentioned, the term "1,2-dimyristoyl-sn-glycero-3-phosphorylglycerol/DMPG" includes, but not limited to, 1,2-dimyristoyl-sn-glycero-3-phosphorylglycerol/DMPG base, 1,2-dimyristoyl-sn-glycero-3-phosphorylglycerol/DMPG sodium, or any other salts thereof.

Unless and otherwise mentioned, the term "N-(carbonyl-methoxypolyethyleneglycol 2000)-1,2-distearoyl-sn-glycero-3-phosphoethanolamine/mPEG₂₀₀₀-DSPE" includes, but not limited to, N-(carbonyl-methoxypolyethyleneglycol 2000)-1,2-distearoyl-sn-glycero-3-phosphoethanolamine/mPEG₂₀₀₀-DSPE base, N-(carbonyl-methoxypolyethyleneglycol 2000)-1,2-distearoyl-sn-glycero-3-phosphoethanolamine/mPEG₂₀₀₀-DSPE sodium, or any other salts thereof.

The stable lyophilized powder of the invention remains stable for commercially relevant time period after manufacturing, such as for about 1, 3, 6, 12, 18, 24 or 36 months, when it is kept in its original packaging under the specified storage conditions.

The reconstituted solution or diluted solution of the present invention which is prepared by mixing lyophilized powder and suitable liquid vehicle remains stable for relevant hold time period, such as for about 1, 3, 6, 8, 12, 18, 24 or 36 hours when it is kept under the specified storage conditions.

The diluted solution of the invention which is prepared by mixing the reconstituted solution and suitable liquid diluent and remains stable for relevant hold time period, such as for about 1, 3, 6, 8, 12, 18, 24 or 36 hours when it is kept under the specified storage conditions.

The term "lyophilizable" means a solution which is able to go under lyophilization process using conventional methods and provides lyophilized powder or cake upon lyophilization.

As used herein, the term "micelle" means an aggregate of surfactant and / or phospholipid molecules, normally spherical in shape, wherein the aggregate have hydrophobic center and hydrophilic periphery.

As used herein, the term "drug-phospholipid micelle" means a micelle formed by drug and phospholipid molecules, wherein drug molecules are present in solubilized form in the hydrophobic core of the micelle.

As used herein, the term "carfilzomib-DMPG micelle" means a micelle, comprising carfilzomib and DMPG molecules, wherein carfilzomib molecules are present in solubilized form in the hydrophobic core of the micelle.

As used herein, the term "carfilzomib-mPEG₂₀₀₀-DSPE micelle" means a micelle, comprising carfilzomib and mPEG₂₀₀₀-DSPE molecules, wherein carfilzomib molecules are present in solubilized form in the hydrophobic core of the micelle.

As used herein, the term "clear solution" means a solution which does not comprise any visible particulate matter, liposome or nanoparticles. The clear solution provides absorbance, when measured at 420 nm, not more than 0.1 AU (absorbance unit), for example, not more than 0.05 AU, not more than 0.04 AU or not more than 0.03 AU. The clear solution provides % transmittance, when measured at 650 nm, not less than 97%, for example, not less than 98%, not less than 99%, not less than 99.5%, not less than 99.6%, not less than 99.7% or not less than 99.8%. The clear solution provides osmolality between 300 mOsmol/kg and 450 mOsmol/kg, for example, about 325 mOsmol/kg, about 350 mOsmol/kg, about 375 mOsmol/kg, about 400 mOsmol/kg or about 425 mOsmol/kg.

As used herein, the term "powder for injection" means powder to prepare liquid composition suitable for parenteral administration. Such liquid composition may be prepared by mixing the powder with one or more of suitable liquid diluents such as water, dextrose, saline and the like.

As used herein, the term "buffer" means a chemical agent able to absorb a certain quantity of acid or base without undergoing a strong variation in the pH.

As used herein, the term "about" refers to encompass +/- 20%, 15%, 10%, 5%, 2%, 1%, 0.5%, or 0.25% of the numerical value of the number with which it is being used.

### Abbreviations:

RT = Room temperature
RH = Relative humidity
1 M, 2 M and 3 M = 1 month, 2 months and 3 months, respectively.
ICH = International Conference on Harmonisation
ND = Not detected
NA = Not analyzed
CRT = Controlled room temperature
BQL = Below quantification level/limit
BDL = Below detection level/limit
HPLC = High performance liquid chromatography
KF titration = Karl Fischer titration
°C = Degree Celsius (unit of temperature)
µ = Micrometer (unit of length)

**IUPAC name and structures of the named impurities**

| **Impurity** | **IUPAC Name of Impurity** | **Structure of Impurity** |
|---|---|---|
| **Diol impurity** | (S)-4-Methyl-N-((S)-1-(((S)-4-methyl-1-((R)-2-methyl-2-hydroxy-2'-hydroxy methyl-2-yl)-1-oxopentan-2-yl)amino)-1-oxo-3-phenylpropan-2-yl)-2-((S)-2-(2-morpholino acetamido)-4-phenyl butanamido) pentanamide | 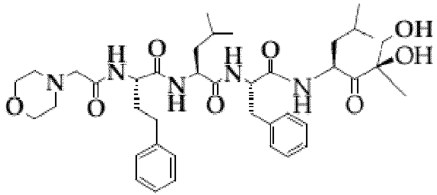 |
| **N-Oxide impurity** | (S)-4-Methyl-N-((S)-1-(((S)-4-methyl-1-((R)-2-methyloxiran-2-yl)-1-oxopentan-2-yl)amino)-1-oxo-3-phenylpropan-2-yl)-2-((S)-2-(2-morpholino-N-oxide acetamido)-4-phenyl butanamido) pentanamide | 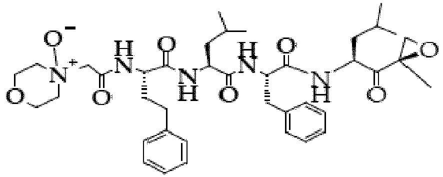 |
| **Epoxy amine impurity** | ((2S)-N-((S)-1-((S)-4-methyl-1-((R)-2-methyloxiran-2-yl)-1-oxopentan-2-ylcarbamoyl)-2-phenylethyl)-2-((S)-amino-4-phenylbutanamido)-4-methylpentanamide | 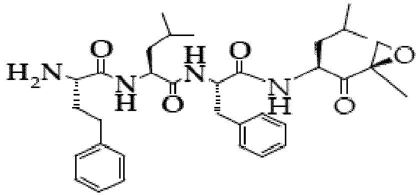 |
| **Epoxy leucine SS impurity** | (S)-4-Methyl-N-((S)-1-(((S)-4-methyl-1-((S)-2-methyloxiran-2-yl)-1-oxopentan-2-yl)amino)-1-oxo-3-phenylpropan-2-yl)-2-((S)-2-(2-morpholino acetamido)-4-phenyl butanamido) pentanamide | 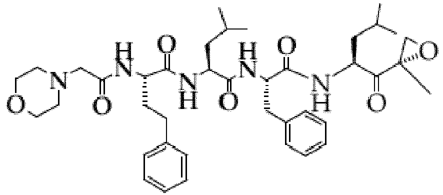 |
| **Epoxy leucine RR impurity** | (S)-4-Methyl-N-((S)-1-(((R)-4-methyl-1-((R)-2-methyloxiran-2-yl)-1-oxopentan-2-yl)amino)-1-oxo-3-phenylpropan-2-yl)-2-((S)-2-(2-morpholinoacetamido) -4-phenylbutanamido) pentanamide | 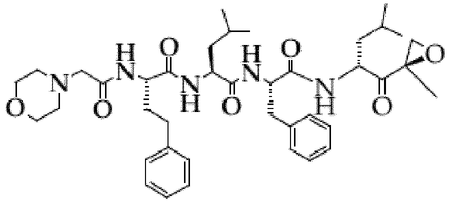 |
| **Alkene impurity** | (S)-4-Methyl-N-((S)-1-(((S)-4-methyl-1-(2-methyl-2-ene-2-yl)-1-oxopentan-2-yl)amino)-1-oxo-3-phenylpropan-2-yl)-2-((S)-2-(2-morpholino acetamido)-4-phenylbutanamido) pentanamide | |
| | | 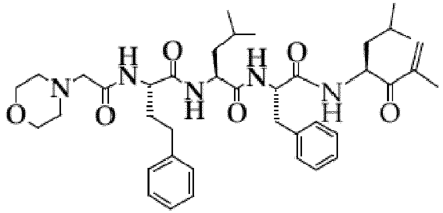 |

The suitable pharmaceutically acceptable excipients for the composition of the present invention may include one or more of the pharmaceutically acceptable solvents, phospholipids, pH adjusting agents, stabilizers, buffers, cryoprotectants, preservatives, isotonicity adjusting agents, surfactants, and anti-oxidants.

Examples of solvents may include, but not limited to, water for injection, organic solvents or mixture of water for injection with one or more organic solvents. The examples of organic solvents are tert-butyl alcohol, dehydrated alcohol, acetonitrile, dimethyl sulfoxide, dimethyl acetamide, dimethylformamide, N-methyl-2-pyrrolidone (NMP), acetone, methanol, benzonitrile, isopropyl alcohol, propylene glycol, polyethylene glycol, glycerine and the like.

Examples of phospholipids may include, but not limited to, phosphatidylcholines such as 1,2-dimyristoyl-sn-glycero-3-phosphocholine (DMPC), 1,2-dioleoyl-sn-glycero-3-phosphocholine (DOPC) and the like; phosphatidylglycerols such as 1,2-dimyristoyl-sn-glycero-3-phosphorylglycerol sodium (DMPG Na), 1,2-dipalmitoyl-sn-glycero-3-phosphorylglycerol sodium (DPPG Na), 1,2-distearoyl-sn-glycero-3-phosphorylglycerol sodium (DSPG Na), 1,2-dioleoyl-sn-glycero-3-phosphorylglycerol sodium (DOPG Na) and the like; phosphatidylserines such as 1,2-dimyristoyl-sn-glycero-3-phosphoserine sodium (DMPS Na), 1,2-dipalmitoyl-sn-glycero-3-phosphoserine sodium (DPPS Na), 1,2-distearoyl-sn-glycero-3-phosphoserine sodium (DSPS Na),1,2-dioleoyl-sn-glycero-3-phosphoserine sodium (DOPS Na) and the like; phosphatidic acids such as 1,2-dimyristoyl-sn-glycero-3-phosphatidic acid sodium (DMPA Na), 1,2-dipalmitoyl-sn-glycero-3-phosphatidic acid sodium (DPPA Na), 1,2-distearoyl-sn-glycero-3-phosphatidic acid sodium (DSPA Na), 1,2-dioleoyl-sn-glycero-3-phosphatidic acid sodium (DOPA Na) and the like; phosphatidylethanolamines such as 1,2-dimaristoyl-sn-glycero-3-phosphoethanolamine (DMPE), 1,2-dioleoyl-sn-glycero-3-phosphoethanolamine (DOPE) and the like; methoxy conjugated phospholipids such as N-(Carbonyl-methoxypolyethyleneglycol 2000)-1,2-distearoyl-sn-glycero-3-phosphoethanolamine sodium (mPEG₂₀₀₀-DSPE Na), N-(Carbonyl-methoxypolyethyleneglycol 5000)-1,2-distearoyl-sn-glycero-3-phosphoethanolamine sodium (mPEG₅₀₀₀-DSPE Na), N-(Carbonyl-methoxypolyethyleneglycol 2000)-1,2-dipalmitoyl-sn-glycero-3-phosphoethanolamine sodium (mPEG₂₀₀₀-DPPE Na), N-(Carbonyl-methoxypolyethyleneglycol 5000)-1,2-dipalmitoyl-sn-glycero-3-phosphoethanolamine sodium (mPEG₅₀₀₀-DPPE Na) and the like, or any combination thereof. The amount of the phospholipid present in the composition may vary and depends on the nature and type of the drug as well as amount of drug to be administered, nature and type of the phospholipid, type of interaction between drug and phospholipid molecules in the aqueous phase, etc. For example, amount of Dimyristoyl phosphatidylglycerol (DMPG) may be such that the solution contains concentration of DMPG between 1 mg/mL and 50 mg/mL. For example, amount of N-(Carbonyl-methoxypolyethyleneglycol 2000)-1,2-distearoyl-sn-glycero-3-phosphoethanolamine sodium (mPEG₂₀₀₀-DSPE Na) may be such that the solution contains concentration of mPEG₂₀₀₀-DSPE between 1 mg/mL and 50 mg/mL.

Examples of pH adjusting agents may include, but not limited to, sodium hydroxide, hydrochloric acid, boric acid, citric acid, acetic acid, phosphoric acid, succinic acid, potassium hydroxide, ammonium hydroxide, magnesium oxide, calcium carbonate, magnesium carbonate, malic acid, potassium citrate, sodium phosphate, lactic acid, gluconic acid, tartaric acid, fumaric acid, diethanolamine, monoethanolamine, sodium carbonate, sodium bicarbonate, triethanolamine, or any combination thereof.

Examples of stabilizers may include, but not limited to, polyvinylpyrrolidone, polyvinyl alcohol, polyethylene glycol, diethanolamine, ferric chloride, inositol, sodium gluconate, sucrose, mannitol, creatinine, glycerine, niacinamide, sodium saccharin, sodium caprylate, arginine, methionine, cysteine and the like, or any combination thereof. Examples of buffers may include, but not limited to, acetate (e.g. sodium acetate etc.), citrate (e.g. citric acid/sodium citrate etc.), phosphate (e.g. monobasic sodium phosphate, dibasic sodium phosphate etc.), carbonate, or any combination thereof.

Examples of cryoprotectants may include, but not limited to, sucrose, lactose, mannitol, polyethylene glycol, polyvinylpyrrolidone, or any combination thereof.

Examples of preservatives may include, but not limited to, chlorobutanol, benzalkonium chloride, methyl paraben, propyl paraben, benzoic acid, sodium benzoate, sorbic acid, benzethonium chloride, cetyl pyridinium chloride, benzyl bromide, benzyl alcohol, phenylmercury nitrate, phenylmercury acetate, thiomersal, merthiolate, chlorhexidine, phenylethyl alcohol, quaternary ammonium chloride, sodium benzoate, sodium propionate, or any combination thereof.

Examples of isotonicity adjusting agents may include, but not limited to, sodium chloride, potassium chloride, calcium chloride, magnesium chloride, glucose, sucrose, dextrose, mannitol, glycerol, or any combination thereof.

Examples of suitable pharmaceutically acceptable surfactants may include, but not limited to, amphoteric, non-ionic, cationic or anionic molecules. Suitable surfactants may include, but not limited to, polysorbates (e.g. tween 80 etc.), poloxamer (poloxamer 188), sodium lauryl sulfate, lauryl dimethyl amine oxide, docusate sodium, cetyl trimethyl ammonium bromide (CTAB), polyethoxylated alcohols, polyoxyethylene sorbitan, octoxynol, polyoxyl lauryl ether, polyoxyethylene vegetable-based fatty ethers derived from lauryl, cetyl, stearyl and oleyl alcohols, bile salts (such as sodium deoxycholate and sodium cholate), polyoxyl castor oil, nonylphenol ethoxylate, lecithin, polyoxyethylene surfactants, polyethylene glycol esters, glycol esters of fatty acids, monoalkanolamine condensates, polyoxyethylene fatty acid amides, quaternary ammonium salts, polyoxyethylene alkyl and alicyclic amines, polyoxyethylene, sorbitan monolaurate and stearate, Cremophor® (polyethoxylated castor oil), Solutol® (ethylene oxide/12-hydroxy stearic acid), tyloxapol, or any combination thereof.

Examples of suitable pharmaceutically acceptable anti-oxidants may include, but not limited to, butylated hydroxytoluene (BHT), butylated hydroxyanisole (BHA), and propyl gallate (PG), monothioglycerol, ascorbic acid, citric acid, tartaric acid, sodium ascorbate, erythorbic acid, potassium metabisulfite, sodium metabisulfite, propionic acid, sodium formaldehyde sulphoxylate, reduced glutathione, thiourea, cysteine, n-acetylcysteine, methionine, sodium sulfite, alkyl gallate, including propyl gallate, vitamin E, or other tocopherol analogs, including tocopherol acetate or TPGS, or any combination thereof.

The sterile injectable compositions of the present invention may have improved physical and/or chemical stability.

The stable lyophilized powder composition of the present invention may retain at least 90% potency (assay) of the drug after storage for more than 3 months, for example, for 6 months, for 12 months, 18 months, 24 months or 36 months, at 2°C - 8°C.

The stable lyophilized powder composition of the present invention may retain at least 90% potency (assay) of the drug after storage for more than 3 months, for example, for 6 months, for 12 months, 18 months, 24 months or 36 months, at 25°C ± 2°C temperature and 60 %RH (relative humidity).

The stable lyophilized powder composition of the present invention may retain at least 90% potency (assay) of the drug after storage for more than 3 months, for example, for 6 months, for 12 months, 18 months, 24 months or 36 months, at 40°C ± 2°C and 75 %RH.

The stable lyophilized powder composition of the present invention may retain at least 90% assay of the mPEG₂₀₀₀-DSPE Na after storage for more than 3 months, for example, for 6 months, for 12 months, 18 months, 24 months or 36 months, at 2°C - 8°C.

The stable lyophilized powder composition of the present invention may retain at least 90% assay of the mPEG₂₀₀₀-DSPE after storage for more than 6 months, for example, for 12 months, 18 months, 24 months or 36 months, at 25°C ± 2°C temperature and 60 %RH.

The stable lyophilized powder composition of the present invention may retain at least 90% assay of the DMPG Na after storage for more than 3 months, for example, for 6 months, for 12 months, 18 months, 24 months or 36 months, at 2°C - 8°C.

The stable lyophilized powder composition of the present invention may retain at least 90% assay of the DMPG Na after storage for more than 6 months, for example, for 12 months, 18 months, 24 months or 36 months, at 25°C ± 2°C temperature and 60 %RH.

The stable, clear, aqueous solution of the present invention may retain at least 90% potency (assay) of the drug after storage for 1 hour or more, for example, storage for 2 hours, 4 hours, 6 hours, 8 hours, 12 hours, 18 hours, 24 hours, 36 hours or 48 hours, at 2°C - 8°C.

The stable, clear, aqueous solution of the present invention may retain at least 90% potency (assay) of the drug after storage for 1 hour or more, for example, storage for 2 hours, 4 hours, 6 hours, 8 hours, 12 hours, 18 hours, 24 hours, 36 hours or 48 hours, at 25°C ± 2°C temperature and 60 %RH.

In one embodiment, there is provided a stable lyophilized powder comprising carfilzomib, which remains stable and compliant as per regulatory requirements for impurities for respective product, for more than 2 months, for example, for 3 months, for 6 months, for 12 months, for 24 months or for 36 months, when stored at 25°C ± 2°C temperature and 60 %RH or at 2°C - 8°C temperature.

In one embodiment, there is provided a stable, clear, aqueous solution comprising carfilzomib, which remains stable and compliant as per regulatory requirements for impurities for respective product, for more than 4 hours, for example, for 8 hours, for 12 hours, for 18 hours, 24 hours or for 36 hours, when stored at 2°C - 8°C temperature or at 25°C ± 2°C temperature and 60 %RH.

In another embodiment of the invention, there is provided a stable lyophilized powder comprising carfilzomib which does not contain more than 1% of diol impurity, does not contain more than 1% of N-Oxide impurity, does not contain more than 1% of epoxy amine impurity, does not contain more than 1% of epoxy leucine SS impurity, does not contain more than 1% of epoxy leucine RR impurity, does not contain more than 1% of alkene impurity, does not contain more than 1% of the single maximum unknown impurity and/or does not contain more than 7% (for example, not more than 6%, 5%, 4%, 3%, 2%, 1% or 0.5%) of the total impurities when stored at 2°C - 8°C temperature or at 25°C ± 2°C temperature and 60 %RH, for more than 2 months, for example, for 3 months, for 6 months, for 12 months, for 18 months, for 24 months or for 36 months.

In another embodiment of the invention, there is provided a stable clear aqueous solution comprising carfilzomib which does not contain more than 1% of diol impurity, does not contain more than 1% of N-Oxide impurity, does not contain more than 1% of epoxy amine impurity, does not contain more than 1% of epoxy leucine SS impurity, does not contain more than 1% of epoxy leucine RR impurity, does not contain more than 1% of alkene impurity, does not contain more than 1% of the single maximum unknown impurity and/or does not contain more than 7% (for example, not more than 6%, 5%, 4%, 3%, 2%, 1% or 0.5%) of the total impurities when stored at 2°C - 8°C temperature or at 25°C ± 2°C temperature, for more than 1 hour, for example, for 2 hours, for 3 hours, for 4 hours, for 5 hours, for 6 hours, for 7 hours, for 8 hours, for 10 hours, for 12 hours, for 18 hours, for 24 hours or for 48 hours. The stable, clear, aqueous solution comprising carfilzomib does not form precipitate and remains physically stable for up to 1 hour, 2 hours, 3 hours, 4 hours, 5 hours, 6 hours, 7 hours, 8 hours, 10 hours, 12 hours, 18 hours, 24 hours, 36 hours or 48 hours when stored at 2°C - 8°C temperature or at 25°C ± 2°C temperature.

In another embodiment of the invention, there is provided a stable lyophilized powder comprising carfilzomib which contain water content, as determined by KF titration, not more than 5 %w/w of the lyophilized powder composition, for example not more than 4 %w/w, not more than 3 %w/w, not more than 2 %w/w or not more than 1 %w/w.

The stable, clear, aqueous solution comprising carfilzomib does not form precipitate and remains physically stable up to 6 hours, 8 hours, 10 hours, 12 hours, 18 hours, 24 hours, 36 hours or 48 hours when stored at 2°C - 8°C temperature or at 25°C ± 2°C temperature.

In one embodiment, the present invention provides a process for preparing the lyophilized powder comprising one or more drugs and one or more phospholipids. The process includes the steps of (a) preparing aqueous solution comprising one or more phospholipids; (b) preparing non-aqueous solution comprising one or more drugs; (c) mixing the solution of step (a) and step (b); and (d) lyophilizing the solution mixture obtained at step (c). So obtained lyophilized powder may be reconstituted using an appropriate quantity of one or more of water for injection, 5% dextrose or normal saline solution (0.9% NaCl), to provide a stable, clear, aqueous reconstituted solution, for parenteral administration. The reconstituted solution may be further diluted using an appropriate quantity of one or more of water for injection, 5% dextrose or normal saline solution (0.9% NaCl), to provide a stable, clear, aqueous diluted solution, for parenteral administration.

In another embodiment, the present invention provides a process for preparing the lyophilized powder comprising carfilzomib and mPEG₂₀₀₀-DSPE. The process includes the steps of (a) preparing aqueous solution comprising mPEG₂₀₀₀-DSPE; (b) preparing non-aqueous solution comprising carfilzomib; (c) mixing the solution of step (a) and step (b); and (d) lyophilizing so obtained solution mixture to obtain lyophilized powder. Additionally, PVP or other stabilizer may be added at step (a), (b) or (c). Additionally, DMPG may be added at step (a). So the powder obtained at step (d) may be reconstituted using an appropriate quantity of one or more of water for injection, 5% dextrose or normal saline solution (0.9% NaCl), to provide a stable, clear, aqueous reconstituted solution, for parenteral administration. The reconstituted solution may be further diluted using an appropriate quantity of one or more of water for injection, 5% dextrose or normal saline solution (0.9% NaCl), to provide a stable, clear, aqueous diluted solution, for parenteral administration.

The following examples are for the purpose of illustration of the invention only and are not intended in any way to limit the scope of the present invention.

### Example 1

**Table 1**

| **Ingredients** | **Quantity/mL** |
|---|---|
| Carfilzomib | 2 mg |
| N-(Carbonyl-methoxypolyethyleneglycol 2000)-1,2-distearoyl-sn-glycero-3-phosphoethanolamine sodium (mPEG₂₀₀₀-DSPE Na) | 5 mg |
| Polyvinylpyrrolidone K-12 | 35 mg |
| Citric acid | 2 mg |
| Dehydrated alcohol | 0.06 mL |
| Sodium Hydroxide | q.s. To pH 3.5 |
| Water for Injection (WFI) | q.s. to 1 mL |

### Process

### Manufacturing steps:

### (a) Aqueous solution

Accurately weighed mPEG₂₀₀₀-DSPE was added to an appropriate quantity of water for injection at room temperature under stirring and continued to stir till it became clear solution.

To the obtained clear solution, accurately weighed Polyvinylpyrrolidone K-12 and Citric acid were added and stirred until dissolved.

### (b) Non-aqueous solution

Accurately weighed carfilzomib was added into the dehydrated alcohol under stirring and continued to stir till it became clear solution.

### (c) Homogeneous solution

Aqueous solution obtained in step (a) was added to the non-aqueous solution obtained in step (b) under stirring and continued to stir till it became clear solution. Volume was adjusted using water for injection.

Thenafter, pH was checked and adjusted using appropriate quantities of sodium hydroxide to have pH of aqueous solution about 3.5.

Thus obtained homogeneous solution was filtered through 0.22µ filter to obtain ready for lyophilization solution.

### (d) Lyophilization

Thus obtained ready for lyophilization solution was filled into 30 mL glass vials, in the required quantity so that each vial contains 30 mg carfilzomib/vial. So obtained glass vials were lyophilized to obtain lyophilized powder composition. In the similar way, lyophilized powder composition containing 60 mg of carfilzomib in a vial may be obtained by lyophilization of a glass vial filled with appropriate required quantity of the homogeneous solution obtained at step (c).

### (e) Reconstitution

Thus obtained lyophilized powder in above step (d) was reconstituted using water for injection to provide reconstituted solution having 2 mg/mL carfilzomib.

### (f) Dilution

Thus obtained reconstituted solution in above step (e) was further diluted using 5% dextrose to provide diluted solution having 0.6 mg/mL carfilzomib.

The ready for lyophilization solution obtained in above mentioned step (c) was tested for its physical and chemical stability and results are reported in the Table 2 and Table 3.

**Table 2 - Physical stability of ready for lyophilization solution, obtained at step (c) of Example 1**

| **Concentration** | **2°C - 8°C** | **CRT** |
|---|---|---|
| 2 mg/mL carfilzomib | Clear solution at 24 hours | Clear solution at 24 hours |

**Table 3 - Chemical stability of ready for lyophilization solution, obtained at step (c) of Example 1**

| **Concentration 2 mg/mL carfilzomib** | **Initial** | **CRT After 24 hours** |
|---|---|---|
| Assay | 97.40 | 97.10 |
| % Total impurities | 0.48 | 0.89 |

### Example 2

Formula and process for the example 2 is similar to the example 1, except the amount of mPEG₂₀₀₀-DSPE was used 6 mg/mL instead of 5 mg/mL.

The ready for lyophilization solution obtained in the step (c), reconstituted solution obtained at step (e) and diluted solution obtained at step (f) of example 2 were tested for its physical stability and results are reported in the Table 4 through Table 6.

**Table 4 - Physical stability of ready for lyophilization solution, obtained at step (c) of Example 2**

| **Concentration** | **2°C - 8°C** | **CRT** |
|---|---|---|
| 2 mg/mL carfilzomib | Clear solution at 48 hours | Clear solution at 48 hours |

**Table 5 - Physical stability of reconstituted solution, obtained at step (e) of Example 2**

| **Reconstitution vehicle** | **Concentration** | **2°C - 8°C** | **CRT** |
|---|---|---|---|
| Water for injection | 2 mg/mL carfilzomib | Clear solution at 24 hours | Clear solution at 24 hours |

**Table 6 - Physical stability of diluted solution, obtained at step (f) of Example 2**

| **Dilution vehicle** | **Concentration** | **2°C - 8°C** | **CRT** |
|---|---|---|---|
| 5% dextrose | 0.6 mg/mL carfilzomib | Clear solution at 48 hours | Clear solution at 48 hours |

### Example 3

**Table 7**

| **Ingredients** | **Quantity/mL** |
|---|---|
| Carfilzomib | 2.00 mg |
| 1,2-Dimyristoyl-sn-glycero-3-phosphorylglycerol sodium (DMPG Na) | 10.00 mg |
| N-(Carbonyl-methoxypolyethyleneglycol 2000)-1,2-distearoyl-sn-glycero-3-phosphoethanolamine sodium (mPEG₂₀₀₀-DSPE Na) | 3.50 mg |
| Polyvinylpyrrolidone K12 (PVP K12) | 40.00 mg |
| Citric acid | 0.35 mg |
| Sucrose | 100.00 mg |
| Dehydrated alcohol | 0.06 mL |
| Sodium Hydroxide | q.s. To pH 4.8 |
| Water for Injection (WFI) | q.s. 1 mL |

### Manufacturing steps:

### (a) Aqueous solution

Accurately weighed DMPG Na was added to an appropriate quantity of water for injection at 50°C under stirring and continued to stir till it became clear solution and allowed to come at room temperature.

To the obtained clear solution, accurately weighed mPEG₂₀₀₀-DSPE, polyvinylpyrrolidone K12, citric acid and sucrose were added and stirred until dissolved.

Thenafter, pH was checked and adjusted using appropriate quantity of sodium hydroxide to have pH of aqueous solution about 4.8.

### (b) Non-aqueous solution

Accurately weighed carfilzomib was added to an appropriate quantity of dehydrated alcohol under stirring and continued to stir till it became clear solution.

### (c) Homogeneous solution

Aqueous solution obtained in step (a) was added to the non-aqueous solution obtained in step (b) under stirring and continued to stir till it became clear solution. Volume was made using water for injection.

Thenafter, pH was checked and adjusted using appropriate quantities of citric acid solution and sodium hydroxide to have pH of aqueous solution about 4.8.

Thus obtained homogeneous solution was filtered through 0.22µ filter to obtain ready for lyophilization solution.

### (d) Lyophilization

Thus obtained ready for lyophilization solution was filled into 30 mL glass vials, in the required quantity so that each vial contains 30 mg carfilzomib/vial. So obtained glass vials were lyophilized to obtain lyophilized powder composition. In the similar way, lyophilized powder composition containing 60 mg of carfilzomib in a vial may be obtained by lyophilization of a glass vial filled with appropriate required quantity of the homogeneous solution obtained at step (c).

### (e) Reconstitution

Thus obtained lyophilized powder in above step (d) was reconstituted using water for injection to provide reconstituted solution having 2 mg/mL carfilzomib.

### (f) Dilution

Thus obtained reconstituted solution in above step (e) was further diluted using 5% dextrose to provide diluted solution having 0.6 mg/mL carfilzomib.

The ready for lyophilization solution obtained in above mentioned step (c) and its diluted solution prepared using 5% dextrose to have 0.6 mg/mL carfilzomib were tested for their physical stability and results are reported in Table 8 and Table 9.

**Table 8 - Physical stability of ready for lyophilization solution, obtained at step (c) of Example 3**

| Concentration | 2°C - 8°C |
|---|---|
| 2 mg/mL carfilzomib | Clear solution at 24 hours |

**Table 9 - Physical stability of diluted solution prepared by diluting the ready for lyophilization solution, obtained at step (c) of Example 3 using 5% dextrose**

| **Dilution vehicle** | **Concentration** | **2°C - 8°C** | **CRT** |
|---|---|---|---|
| 5% dextrose | 0.6 mg/mL carfilzomib | Clear solution at 24 hours | Clear solution at 24 hours |

### Example 4

**Table 10**

| **Ingredients** | **Quantity/mL** |
|---|---|
| Carfilzomib | 4 mg |
| 1,2-Dimyristoyl-sn-glycero-3-phosphorylglycerol sodium (DMPG Na) | 0.7 mg |
| N-(Carbonyl-methoxypolyethyleneglycol distearoyl-sn-glycero-3-phosphoethanolamine (mPEG₂₀₀₀-DSPE Na) 2000)-1,2-sodium | 12 mg |
| Polyvinylpyrrolidone K12 (PVP K12) | 34 mg |
| Anhydrous citric acid | 1.7 mg |
| Sucrose | 50 mg |
| Dehydrated alcohol (Absolute Ethanol) | 0.06 mL |
| Sodium Hydroxide | q.s. To pH 3.5 |
| Water for Injection (WFI) | q.s. 1 mL |

### Manufacturing steps:

### (a) Aqueous solution

Water for injection (80% quantity of the total batch size) was collected in clean glass bottle and kept under nitrogen purging until dissolved oxygen level was below 2 ppm.

Accurately weighed DMPG Na was added to the water for injection at 50°C - 60°C under stirring and continued to stir till it became clear solution and then allowed to come at room temperature.

To the obtained clear solution, accurately weighed mPEG₂₀₀₀-DSPE Na, polyvinylpyrrolidone K12, anhydrous citric acid and sucrose were added and stirred until dissolved.

### (b) Non-aqueous solution

Accurately weighed carfilzomib was added to an appropriate quantity of dehydrated alcohol (absolute ethanol) at 40°C - 45°C under stirring and continued to stir till it became clear solution.

### (c) Homogeneous solution

Non-aqueous solution obtained in step (b) was added to the aqueous solution obtained in step (a) under stirring and continued to stir till it became clear solution. Volume was made using water for injection.

Thenafter, pH was checked and adjusted using appropriate quantity of sodium hydroxide (1% w/v) to have pH of aqueous solution about 3.5.

Thus obtained homogeneous solution was filtered through 0.22µ filter to obtain ready for lyophilization solution.

### (d) Lyophilization

Thus obtained ready for lyophilization solution was filled into 10 mL glass vials, in the required quantity so that each vial contains 10 mg carfilzomib/vial. So obtained glass vials were lyophilized to obtain lyophilized powder composition. In the similar way, appropriate required quantities of the homogeneous solution obtained at step (c) were filled into 30 mL glass vials or 50 mL glass vials and lyophilized to obtain lyophilized powder composition containing 30 mg of carfilzomib/vial or 60 mg of carfilzomib/vial, respectively.

### (e) Reconstitution

Thus obtained lyophilized powder in above step (d) was reconstituted using appropriate quantities of 5% dextrose to provide reconstituted solution having 2 mg/mL carfilzomib.

### (f) Dilution

Thus obtained reconstituted solution in above step (e) was further diluted using 5% dextrose to provide diluted solution having 0.27 mg/mL carfilzomib.

The lyophilized powder composition obtained in the step (d) of example 4 was tested for physical stability and results are reported in Table 11.

**Table 11 - Physical stability of lyophilized powder composition, obtained at step (d) of Example 4**

| **Test Parameters** | **10 mg/vial** | **30 mg/vial** | **60 mg/vial** |
|---|---|---|---|
| Description | A white lyophilized cake | A white lyophilized cake | A white lyophilized cake |
| Reconstitution time (Unit: minutes) | 6 | 6 | 6 |
| Water content as determined by KF titration (Unit: %w/w of the lyophilized powder composition) | 0.26 | 0.18 | 0.16 |
| Assay of carfilzomib by HPLC (Unit:% of label amount) | 102.80 | 100.70 | 101.60 |
| Assay of mPEG₂₀₀₀-DSPE Na by HPLC (Unit:% of label amount) | 102.40 | 98.20 | 95.30 |
| Assay of DMPG Na by HPLC (Unit:% of label amount) | 103.10 | 93.80 | 94.10 |
| Diol impurity | BQL | BQL | BQL |
| N-Oxide impurity | BQL | BQL | BQL |
| Epoxy amine impurity | ND | ND | ND |
| Epoxy leucine SS impurity | ND | ND | ND |
| Epoxy leucine RR impurity | 0.10 | 0.09 | 0.10 |
| Alkene impurity | BQL | BQL | BQL |
| Any unspecified impurity | 0.07 | 0.07 | 0.07 |
| Total impurities | 0.30 | 0.30 | 0.30 |

The reconstituted solutions, obtained at step (e) were tested for their physical stability and results are reported in Table 12 and Table 13.

**Table 12 - Physical stability of reconstituted solution (2 mg/mL), obtained at step (e) of Example 4**

| **Test Parameters** | **10 mg/vial** | **30 mg/vial** | **60 mg/vial** |
|---|---|---|---|
| Appearance after reconstitution | Clear colorless solution. | Clear colorless solution. | Clear colorless solution. |
| pH of reconstituted solution | 3.50 | 3.40 | 3.40 |
| Clarity test (% Transmittance at 650 nm) | 98.40 | 99.60 | 99.90 |
| Color test (Absorbance at 420 nm in AU) | 0.02 | 0.02 | 0.02 |
| Osmolality (Unit: mOsmol/kg) | 397 | 396 | 401 |

The 10 mg carfilzomib/vial lyophilized powder composition obtained in the step (d) of example 4 was stored at 2°C - 8°C temperature and also at 25°C ± 2°C temperature and 60 %RH ± 5%RH for 3 months as mentioned in Table 13. After 3 months of the storage, 10 mg carfilzomib/vial lyophilized powder composition was tested for physical and chemical stability and results are reported in Table 13.

**Table 13 - Physical and chemical stability of lyophilized powder composition, obtained at step (d) of Example 4 after storage for 3 months at different storage conditions**

| **Test Parameters** | **Storage conditions (2°C - 8°C) (3M)** |
|---|---|
| Description | White lyophilized cake |
| Reconstitution time (Unit: minutes) | 6 |
| Water content as determined by KF titration (Unit: %w/w of the lyophilized powder composition) | 0.36 |
| Assay of Carfilzomib by HPLC (Unit:% of label amount) | 97.30% |
| Diol impurity | BQL |
| N-Oxide impurity | BQL |
| Epoxy amine impurity | BQL |
| Epoxy leucine SS impurity | ND |
| Epoxy leucine RR impurity | 0.12 |
| Alkene impurity | ND |
| Any unspecified impurity | 0.12 |
| Total impurities | 0.40 |

The 10 mg carfilzomib/vial lyophilized powder composition obtained in the step (d) of example 4 was stored at 2°C - 8°C temperature and also at 25°C ± 2°C temperature and 60 %RH ± 5%RH for 3 months as mentioned in Table 14. After 3 months of said storage, 10 mg carfilzomib/vial lyophilized powder composition was reconstituted using 5% dextrose solution to provide 2 mg/mL carfilzomib reconstituted solution. Thus obtained reconstituted solution was tested for its physical stability and results are reported in Table 14.

**Table 14**

| **Test Parameters** | **Storage conditions (3M)** | |
|---|---|---|
| | Storage conditions (2°C - 8°C) | 25°C ± 2°C temperature and 60 %RH |
| Appearance after reconstitution | Clear colourless solution. | Clear colourless solution. |
| pH of reconstituted solution | 3.40 | 3.40 |
| Clarity test (% Transmittance at 650 nm) | 98.65 | 98.46 |
| Color test (Absorbance at 420 nm in AU) | 0.02 | 0.02 |
| Osmolality (Unit: mOsmol/kg) | 401 | 399 |

The 10 mg carfilzomib/vial lyophilized powder composition obtained in the step (d) of example 4 was reconstituted using 5% dextrose solution to provide 2 mg/mL carfilzomib reconstituted solution in a vial. The reconstituted solution was stored for relevant period of hold time in the vial, tested for its physical and chemical stability, and results are reported in Table 15.

**Table 15**

| **Test Parameters** | **Storage conditions** | | | |
|---|---|---|---|---|
| | 25°C ± 2°C temperature | | | 2°C - 8°C |
| | Initial | 4 hours | 22 hours | 24 hours |
| Appearance after reconstitution | Clear colourless solution. | Clear colourless solution. | Clear colourless solution. | Clear colourless solution. |
| pH of reconstituted solution | 3.50 | 3.50 | 3.50 | 3.50 |
| Clarity test (% Transmittance at 650 nm) | 98.40 | 98.10 | 98.80 | 98.20 |
| Color test (Absorbance at 420 nm in AU) | 0.02 | 0.02 | 0.01 | 0.03 |
| Osmolality (Unit: mOsmol/kg) | 397 | 395 | 393 | 386 |
| Assay of Carfilzomib by HPLC (Unit:% of label amount) | 10.38 mg (103.8%) | 10.38 mg (103.8%) | 10.35 mg (103.5%) | 10.39 mg (103.9%) |
| Diol impurity | BQL | BQL | 0.06 | BQL |
| N-Oxide impurity | BQL | BQL | BQL | BQL |
| Epoxy amine impurity | ND | ND | ND | ND |
| Epoxy leucine SS impurity | ND | ND | ND | ND |
| Epoxy leucine RR impurity | 0.09 | 0.09 | 0.10 | 0.10 |
| Alkene impurity | BQL | BQL | BQL | BQL |
| Any unspecified impurity | 0.07 | 0.06 | 0.10 | 0.06 |
| Total impurities | 0.20 | 0.20 | 0.40 | 0.20 |

The 10 mg carfilzomib/vial lyophilized powder composition obtained in the step (d) of example 4 was reconstituted using 5% dextrose solution to provide 2 mg/mL carfilzomib reconstituted solution in a vial. The reconstituted solution was transferred in a polypropylene syringe, stored for relevant period of hold time, tested for its physical stability, and results are reported in Table 16.

**Table 16**

| **Test Parameters** | **Storage conditions** | | | |
|---|---|---|---|---|
| | 25°C ± 2°C temperature and 60 %RH | | | 2°C - 8°C |
| | Initial | 4 hours | 22 hours | 24 hours |
| Appearance after reconstitution | Clear colourless solution. | Clear colourless solution. | Clear colourless solution. | Clear colourless solution. |
| pH of reconstituted solution | 3.5 | 3.5 | 3.5 | 3.5 |
| Clarity test (% Transmittance at 650 nm) | 98.30 | 99.30 | 99.60 | 99.10 |
| Color test (Absorbance at 420 nm in AU) | 0.03 | 0.03 | 0.02 | 0.03 |
| Osmolality (Unit: mOsmol/kg) | 387 | 398 | 385 | 398 |
| Assay of Carfilzomib by HPLC (Unit:% of label amount) | 10.37 mg (103.7%) | 10.30 mg (103.0%) | 10.26 mg (102.6%) | 10.32 mg (103.2%) |
| Diol impurity | BQL | BQL | 0.06 | BQL |
| N-Oxide impurity | BQL | BQL | BQL | BQL |
| Epoxy amine impurity | ND | ND | ND | ND |
| Epoxy leucine SS impurity | ND | ND | ND | ND |
| Epoxy leucine RR impurity | 0.10 | 0.10 | 0.10 | 0.10 |
| Alkene impurity | BQL | BQL | BQL | BQL |
| Any unspecified impurity | 0.07 | 0.06 | 0.10 | 0.06 |
| Total impurities | 0.20 | 0.20 | 0.40 | 0.20 |

The diluted solution, obtained at step (f) of the example 4 was tested for its physico-chemical stability and results are reported in Table 17.

**Table 17 - Physico-chemical stability of Carfilzomib for Injection 0.27 mg/mL in bag**

| **Test Parameters** | **25°C ± 2°C temperature** | | | **2°C - 8°C** | |
|---|---|---|---|---|---|
| | **Initial** | **4 hours** | **22 hours** | **Initial** | **24 hours** |
| Description of diluted solution | Clear Colourless solution | Clear Colourless solution | Clear Colourless solution | Clear Colourless solution | Clear Colourless solution |
| pH of Diluted solution | 3.600 | 3.600 | 3.500 | 3.600 | 3.700 |
| Clarity test (% Transmittance at 650 nm) | 99.900 | 99.900 | 99.800 | 99.700 | 99.500 |
| Color test (Absorbance at 420 nm in AU) | 0.003 | 0.004 | 0.004 | 0.003 | 0.003 |
| Osmolality (Unit: mOsmol/kg) | 281 | 281 | 281 | 284 | 281 |
| Assay of | 106.500 | 107.700 | 106.800 | 97.300 | 96.800 |
| Carfilzomib by HPLC (Unit:% of label amount) | | | | | |
| Related substances (Unit: %) | | | | | |
| Diol impurity | BQL | 0.05 | 0.14 | BQL | BQL |
| N -Oxide impurity | 0.05 | 0.05 | 0.15 | BQL | BQL |
| Epoxy amine impurity | BQL | BQL | BQL | BQL | BQL |
| Epoxy leucine SS impurity | ND | ND | ND | ND | ND |
| Epoxy leucine RR impurity | 0.140 | 0.150 | 0.140 | 0.140 | 0.070 |
| Alkene impurity | BQL | BQL | BQL | BQL | BQL |
| Any unspecified impurity | 0.090 | 0.090 | 0.220 | 0.070 | 0.070 |
| Total impurities | 0.400 | 0.700 | 1.000 | 0.300 | 0.300 |

While the present invention has been described in terms of its specific embodiments, certain modifications and equivalents will be apparent to those skilled in the art and are intended to be included within the scope of the present invention.

## Claims

1. A sterile injectable composition comprising carfilzomib and one or more phospholipids.

2. The sterile injectable composition according to claim 1, wherein the phospholipid is 1, 2-dimyristoyl-sn-glycero-3-phosphorylglycerol (DMPG), N-(carbonyl-methoxypolyethyleneglycol 2000)-1,2-distearoyl-sn-glycero-3-phosphoethanolamine (mPEG₂₀₀₀-DSPE), or any combination thereof.

3. The sterile injectable composition according to claim 2 further comprises one or more stabilizers.

4. The sterile injectable composition according to claim 3, wherein the stabilizer is polyvinylpyrrolidone (PVP).

5. The sterile injectable composition according to claim 1, wherein the composition is a clear aqueous solution.

6. The sterile injectable composition according to claim 5, wherein the solution further comprises one or more pH adjusting agents to provide pH of the solution about 3.5.

7. The sterile injectable composition according to claim 5, wherein the solution has % transmittance not less than 99%, when measured at 650 nm.

8. The sterile injectable composition according to claim 5, wherein the solution has absorbance not more than 0.1 AU (absorbance unit), when measured at 420 nm.

9. The sterile injectable composition according to claim 5, wherein the solution does not form any precipitate after storage for 24 hours at 2°C - 8°C.

10. The sterile injectable composition according to claim 5, wherein the solution retain at least 90% potency of the carfilzomib after storage for 24 hours at 2°C - 8°C.

11. The sterile injectable composition according to claim 5, wherein the solution does not contain more than 1 % of the total impurities after storage for 24 hours at 2°C - 8°C.

12. The sterile injectable composition according to claim 1, wherein the composition is a lyophilized powder for injection.

13. The sterile injectable composition according to claim 12, wherein the lyophilized powder retain at least 90% potency of the carfilzomib after storage for 12 months at 2°C - 8°C.

14. The sterile injectable composition according to claim 12, wherein the lyophilized powder does not contain more than 1 % of total impurities after storage for 3 months at 2°C - 8°C.

15. The sterile injectable composition according to claim 12, wherein the lyophilized powder provides a clear solution when reconstituted with one or more aqueous vehicles.
